Europäisches Patentamt

European Patent Office

Office européen des brevets

(11) Veröffentlichungsnummer: **0 286 981 B1**

## (12) EUROPÄISCHE PATENTSCHRIFT

(45) Veröffentlichungstag der Patentschrift: **03.02.93**

(51) Int. Cl.⁵: **C07C  67/08**

(21) Anmeldenummer: **88105580.0**

(22) Anmeldetag: **08.04.88**

(54) Verfahren zur Herstellung von Carbonsäuremethylestern.

(30) Priorität: **15.04.87 DE 3712835**

(43) Veröffentlichungstag der Anmeldung:
**19.10.88 Patentblatt  88/42**

(45) Bekanntmachung des Hinweises auf die
Patenterteilung:
**03.02.93 Patentblatt  93/05**

(84) Benannte Vertragsstaaten:
**DE FR GB NL**

(56) Entgegenhaltungen:
**EP-A- 0 072 218**
**DE-A- 3 607**
**DE-C- 1 007 758**

**DE - A - 33607 II d (F. DEON)**

(73) Patentinhaber: **HOECHST AKTIENGESELL-
SCHAFT**
**Postfach 80 03 20**
**W-6230 Frankfurt am Main 80(DE)**

(72) Erfinder: **Weber, Jürgen, Dr. Dipl.-Chem.**
**Bunsenstrasse 17**
**W-4200 Oberhausen 11(DE)**
Erfinder: **Lappe, Peter, Dr. Dipl.-Chem.**
**Eickenhof 34**
**W-4220 Dinslaken(DE)**
Erfinder: **Springer, Helmut, Dipl.-Ing.**
**Drostenkampstrasse 34**
**W-4200 Oberhausen 11(DE)**

**Beschreibung**

Die Erfindung betrifft ein Verfahren zur Herstellung von Carbonsäuremethylestern aus Monocarbonsäuren mit 1 bis 5 C-Atomen im Molekül und Methanol. Hierzu werden die Reaktanten bei Temperaturen oberhalb des Siedepunktes des Esters, mindestens aber etwa 110°C in ein Gemisch aus einem Wärmeträger und dem Katalysator geleitet.

Der gängigste Prozeß zur Herstellung von Carbonsäureestern ist die unmittelbare Umsetzung von Carbonsäuren mit Alkoholen. Sie führt zu einem Gleichgewicht, in dem nebeneinander Alkohol, Säure, Ester und Wasser vorliegen, dessen Einstellung durch Katalysatoren beschleunigt wird.

Diese Arbeitsweise ist in der Regel nur dann wirtschaftlich, wenn das Veresterungsgleichgewicht möglichst weit zugunsten der Esterbildung verschoben wird. Geeignete Maßnahmen, dieses Ziel zu erreichen, sind die Anwendung eines großen Überschusses eines der Ausgangsstoffe oder die Entfernung eines der Produkte aus dem Reaktionsgemisch. Üblicherweise setzt man den Alkohol im Überschuß ein oder man entfernt das Reaktionswasser oder den Ester aus dem Gleichgewicht.

Die Abtrennung des Reaktionswassers kann z.B. durch Zusatz eines Trockenmittels, das das Wasser bindet oder bevorzugt durch azeotrope Destillation mit Hilfe von Schleppmitteln erfolgen.

Bei dem Verfahren der extraktiven Veresterung wird der gebildete Ester mit Hilfe eines Lösungsmittels selektiv aus dem Reaktionsgemisch herausgelöst.

Zur Herstellung von Carbonsäuremethylestern ist die Entfernung des Reaktionswassers durch azeotrope Destillation nicht geeignet. Methanol destilliert nämlich mit dem Schleppmittel in solcher Menge über, daß keine Phasentrennung im Wasserabscheider erfolgt.

Nach einem in J. Org. Chem. 24, 262 (1959) beschriebenen Verfahren gewinnt man Carbonsäuremethylester durch Umsetzung von Carbonsäuren mit Methanol in Gegenwart von Acetondimethylacetal. Das Dimethylacetal wirkt hierbei als wasserbindendes Mittel und als Methanollieferant. Setzt man Adipinsäure, Methanol und Acetondimethylacetal im molaren Verhältnis von 4 : 5 : 8 um, so erhält man Adipinsäuredimethylester in 94 %iger Ausbeute. Die Wirtschaftlichkeit dieser Arbeitsweise wird dadurch beeinträchtigt, daß die Verwendung von Acetondimethylacetal die Rohstoffkosten steigert und die Bildung von Aceton im Verlauf der Reaktion den Destillationsaufwand erhöht.

Nach einer Vorschrift in J. Amer. Chem. Soc. 64, 1801-1803 (1942) stellt man 2-Ethylbuttersäuremethylester durch Umsetzung von 1 Mol 2-Ethylbuttersäure und 7 Mol Methanol in Gegenwart von konzentrierter Schwefelsäure her. Die Esterausbeute beträgt trotz großem Methanol-Überschuß nur 75 %.

Statt organischer und/oder anorganischer Säuren können bei der Veresterung von Carbonsäuren mit Methanol auch Ionenaustauscher in der aciden Form als Katalysatoren eingesetzt werden. So erhält man nach einer Vorschrift in J. Org. Chem. 36, (17), 2548-2550 (1971) Essigsäuremethylester aus Essigsäure und Methanol im Molverhältnis von 1 : 10 in Gegenwart eines Kationenaustauschers und $CaSO_4$ als Trockenmittel.

In der DE-OS 20 50 678 wird die Herstellung von Estern aus Carbonsäuren und Alkoholen in Gegenwart inerter organischer Lösungsmittel und Veresterungskatalysatoren beschrieben. Hierbei verwendet man als inerte organische Lösungsmittel Kohlenwasserstoffe mit einem Siedepunkt von -10 bis 200°C und als Katalysatoren Säuren oder Stoffe, die im wäßrigen Medium Säuren bilden, zusammen mit wäßrigen Salzlösungen. Nach den Beispielen erfordert die Herstellung von Methylestern gesättigter Carbonsäuren Reaktionszeiten von 10 Stunden (Propionsäure) bis 60 Stunden (Valeriansäure), wobei die Esterausbeuten nur 68 % bzw. 66 % betragen. Die langen Reaktionszeiten, der Einsatz von Kohlenwasserstoffen als Extraktionsmittel und die unbefriedigenden Ausbeuten stehen einer Anwendung dieser Verfahrensvariante entgegen.

Aus der US-PS 20 29 694 ist es bekannt, aliphatische Ester von Carbonsäuren in der Weise herzustellen, daß man den Alkohol in die zu veresternde Säure bei Temperaturen einleitet, die etwa zwischen dem Siedepunkt des Esters und etwa 20°C unterhalb dieses Siedepunktes liegen. Das Verfahren ist auf Ester beschränkt, die oberhalb 120°C, aber nicht wesentlich weniger als 50° unterhalb des Siedepunktes der zugehörigen Säure sieden. Vorzugsweise ist es auf Säuren anwendbar, die wenig flüchtig sind und als Anhydride oder dehydratisiert vorliegen. Die nach diesem Verfahren erzielbaren Ester-Ausbeuten liegen jedoch nur wenig über 80 %, bezogen auf eingesetzte Säure.

Der Erfindung liegt die Aufgabe zugrunde, die aufgezeigten Nachteile des Standes der Technik zu vermeiden und ein Verfahren zur Herstellung von Carbonsäuremethylestern zu entwickeln, das bei einfacher Reaktionsführung die Gewinnung der reinen Verbindungen unter wirtschaftlichen Bedingungen und in hoher Ausbeute ermöglicht.

Diese Aufgabe wird gelöst durch ein Verfahren zur Herstellung von Carbonsäuremethylestern aus gesättigten oder ungesättigten, geradkettigen oder verzweigten Monocarbonsäuren mit 1 bis 5 Kohlenstoffatomen im Molekül und Methanol. Es ist dadurch gekennzeichnet, daß man Carbonsäure und Methanol im Molverhältnis von 1 : 1 bis 1 : 5 getrennt oder als Mischung bei Temperaturen oberhalb des Siedepunktes des resultierenden Esters, mindestens aber bei 110°C in ein Gemisch aus einem sauren Katalysator und einem unter den Reaktionsbedingungen flüssigen, gegenüber den Reaktanten und den Reaktionsprodukten inerten Wärmeträger unter Einhaltung einer Verweilzeit von 0,1 bis 5, insbesondere 0,5 bis 2 Stunden leitet.

Obgleich die Reaktionstemperatur deutlich oberhalb der Siedetemperatur des Methanols liegt, gelingt es nach dem erfindungsgemäßen Verfahren überraschenderweise, Carbonsäuremethylester bei einfacher Reaktionsführung und mit einem begrenzten Methanolüberschuß in sehr hohen Ausbeuten leicht herzustellen.

Als Ausgangsstoffe können nach dem erfindungsgemäßen Verfahren geradkettige oder verzweigte, gesättigte oder ungesättigte aliphatische Monocarbonsäuren mit 1 bis 5 Kohlenstoffatomen eingesetzt werden. Beispiele für solche Säuren sind Essigsäure, Propionsäure, n-Buttersäure, Isobuttersäure, 2-Methylbuttersäure, Pivalinsäure, Acrylsäure. Die Carbonsäuren können als einheitliche Verbindung eingesetzt werden. Es lassen sich aber auch Gemische aus zwei oder mehr Verbindungen als Ausgangsstoffe verwenden.

Als Veresterungskatalysatoren eignen sich vornehmlich starke ein- oder mehrbasische, nicht flüchtige anorganische oder organische Säuren, wie Schwefelsäure, Phosphorsäure, Methansulfonsäure, Ethansulfonsäure, Chlorschwefelsäure, p-Toluolsulfonsäure, aber auch saure Salze wie Natriumhydrogensulfat. Besonders geeignet ist konzentrierte Schwefelsäure. Es ist nicht erforderlich, daß sich der Katalysator in dem Wärmeträger vollständig löst. Er kann in ihm auch als weitgehend selbständige, heterogene Phase vorliegen.

Ein charakteristisches Merkmal des erfindungsgemäßen Verfahrens ist die Verwendung eines Wärmeträgers, der die Reaktanten auf die erforderliche Reaktionstemperatur erhitzt. Als Wärmeträger werden gegenüber den Reaktanten, dem Katalysator und den Reaktionsprodukten unter den Reaktionsbedingungen inerte, flüssige Substanzen verstanden. Ihre Siedetemperatur muß deutlich höher liegen als die Siedetemperatur der zu veresternden Säure oder, bei Vorliegen eines Säuregemisches, als der Siedepunkt der höchstsiedenden Säure. Siedepunktdifferenzen zwischen Säure und Wärmeträger von 50 und mehr °C haben sich bewährt. Geeignete Wärmeträger sind Biphenyl, 1- oder 2-Methylnaphthalin und Diphenylmethan, besonders bewährt hat sich Biphenyl.

Die Umsetzung von Säure und Methanol erfolgt bei Temperaturen, die mindestens etwa 10°C oberhalb des Siedepunktes des aus der Reaktion resultierenden Esters liegt und mindestens etwa 110°C beträgt. Bewährt hat es sich, Temperaturen von 10 bis 100°C oberhalb des Ester-Siedepunktes einzuhalten. Bei Einsatz von Säuregemischen richtet sich die Reaktionstemperatur nach dem Siedepunkt des höchstsiedenden Esters.

Die Reaktanten können in äquimolaren Mengen eingesetzt werden. Häufig empfiehlt es sich jedoch, mit einem Methanolüberschuß zu arbeiten und auf 1 Mol Carbonsäure bis zu 5 Mol Methanol einwirken zu lassen.

Bevorzugt wird ein Molverhältnis von Carbonsäure zu Methanol von 1 : 1,75 bis 1 : 3. Zweckmäßig hält man Verweilzeiten von 0,1 bis 5 und insbesondere von 0,5 bis 2 Stunden ein.

Zur praktischen Durchführung der neuen Arbeitsweise legt man in einem Reaktor den inerten Wärmeträger und den gelösten oder suspendierten Katalysator vor und dosiert über eine Zulaufleitung oder, bei getrennter Zuführung, über zwei Leitungen Carbonsäure und Methanol kontinuierlich zu. Unter den angewandten Reaktionsbedingungen destilliert ebenfalls kontinuierlich ein Gemisch aus Carbonsäuremethylester, Methanol und Wasser ab. Das Gemisch wird zur Entfernung des Methanols mit Wasser gewaschen. Darauf trennt man die Methanol enthaltende Wasserphase ab; die organische Phase wird anschließend fraktioniert destilliert.

Die Ausbeuten an Carbonsäuremethylester sind sehr hoch. So erhält man z.B. Pivalinsäuremethylester bei Einsatz von 2,5 Mol Methanol je Mol Pivalinsäure bei 120°C und einer Verweilzeit von 0,96 Stunden in 98,9 %iger Ausbeute.

Die nach dem erfindungsgemäßen Verfahren erhaltenen Produkte sind farblos und weisen Carbonsäuremethylester-Gehalte von mehr als 99 % auf.

Das erfindungsgemäße Verfahren kann sowohl absatzweise als insbesondere auch kontinuierlich durchgeführt werden.

In den nachfolgenden Beispielen wird die neue Arbeitsweise näher erläutert.

Beispiel 1

Herstellung von Pivalinsäuremethylester

EP 0 286 981 B1

In einem 1 l-Kolben, der mit Rührer, Innenthermometer und einer 12 cm Vigreux-Kolonne mit aufgesetzter Claisenbrücken ausgestattet ist, werden 200 g Biphenyl und 5,0 g konzentrierte Schwefelsäure vorgelegt und auf 120°C erwärmt. Unter Rühren wird innerhalb von 66,12 Stunden stündlich ein Gemisch aus 94,2 g (0,92 mol) Pivalinsäure und 73,9 g (2,31 mol) Methanol kontinuierlich in den Reaktionskolben eindosiert. Insgesamt werden 11.114,2 g des Gemisches eingesetzt. Im gleichen Zeitraum destillieren 10.964,2 g Produkt über, das homogene Destillat hat folgende Zusammensetzung:

| Methanol | 26,9 % |
|---|---|
| Pivalinsäuremethylester | 62,9 % |
| Pivalinsäure | 0,2 % |
| Wasser | 9,9 % |
| Sonstige | 0,1 % |

Im Reaktor verbleiben (außer Wärmeträger und Katalysator, das gilt für alle Beispiele) 150,0 g Sumpfprodukt, das 114,5 g Pivalinsäureester enthält. Die Gesamtausbeute an Pivalinsäuremethyleter beträgt 98,9 % der Theorie.

Beispiele 2-4

Herstellung von Pivalinsäuremethylester

Pivalinsäure und Methanol werden, wie in Beispiel 1 beschrieben, jedoch unter Variation des molaren Verhältnisses der Reaktanten, zu Pivalinsäuremethylester umgesetzt. Die Ergebnisse sind in Tabelle 1 zusammengefaßt.

Beispiele 5-7

Herstellung von Pivalinsäuremethylester

Pivalinsäure und Methanol werden, wie in Beispiel 1 beschrieben, jedoch bei unterschiedlichen Reaktionstemperaturen, zu Pivalinsäuremethylester umgesetzt. Die Ergebnisse sind in Tabelle 2 zusammengefaßt.

Beispiele 8 und 9

Herstellung von Pivalinsäuremethylester

Pivalinsäure und Methanol werden, wie in Beispiel 1 beschrieben, jedoch bei unterschiedlichen Durchsatzgeschwindigkeiten, zu Pivalinsäuremethylester umgesetzt. Die Ergebnisse sind in Tabelle 3 zusammengestellt.

Beispiele 10-12

Herstellung von Pivalinsäuremethylester

Pivalinsäure und Methanol werden, wie in Beispiel 1 beschrieben, jedoch unter Variation der eingesetzten Katalysatoren, zu Pivalinsäuremethylester umgesetzt. Die Ergebnisse sind in Tabelle 4 zusammengestellt.

Beispiel 13

Herstellung von Essigsäuremethylester

Entsprechend Beispiel 1 wird in einen Kolben zu 200 g Biphenyl und 5,0 g konzentrierte Schwefelsäure stündlich ein Gemisch aus 60,1 g (1 mol) Essigsäure und 80,1 g (2,5 mol) Methanol bei einer Reaktionstemperatur von 120°C eindosiert. Insgesamt werden innerhalb von 6 Stunden 841,2 g Ausgangsstoffgemisch eingesetzt. Im gleichen Zeitraum destillieren 833,6 g Produkt über, das homogene Destillat hat

4

folgende Zusammensetzung:

| Methanol | 35,3 % |
|---|---|
| Essigsäuremethylester | 51,6 % |
| Essigsäure | 0,3 % |
| Wasser | 12,6 % |
| Sonstige | 0,2 % |

Im Reaktor verbleiben 7,6 g Sumpfprodukt. Die Ausbeute an Essigsäuremethylester beträgt 96,9 % der Theorie.

Beispiel 14

Herstellung von Propionsäuremethylester

Entsprechend Beispiel 1 wird in einen Kolben zu 200 g Biphenyl und 5,0 g konzentrierte Schwefelsäure stündlich ein Gemisch aus 74,0 g (1 mol) Propionsäure und 80,1 g (2,5 mol) Methanol bei einer Reaktionstemperatur von 120°C eindosiert. Insgesamt werden innerhalb von 7 Stunden 1078,7 g Ausgangsstoffgemisch eingesetzt. Im gleichen Zeitraum destillieren 1069,3 g Produkt über, das homogene Destillat hat folgende Zusammensetzung:

| Methanol | 32,0 % |
|---|---|
| Propionsäuremethylester | 56,2 % |
| Propionsäure | 0,2 % |
| Wasser | 11,5 % |
| Sonstige | 0,1 % |

Im Reaktor verbleiben 9,4 g Sumpfprodukt. Die Ausbeute an Propionsäuremethylester beträgt 97,4 % der Theorie.

Beispiel 15

Herstellung von Buttersäuremethylester

Entsprechend Beispiel 1 wird in einen Kolben zu 200 g Biphenyl und 5,0 g konzentrierte Schwefelsäure stündlich ein Gemisch aus 88,0 g (1 mol) Buttersäure und 80,1 g (2,5 mol) Methanol bei einer Reaktionstemperatur von 120°C eindosiert. Insgesamt werden innerhalb von 7 Stunden 1176,7 g Ausgangsstoffgemisch eingesetzt. Im gleichen Zeitraum destillieren 1124,9 g Produkt über, das homogene Destillat hat folgende Zusammensetzung:

| Methanol | 30,0 % |
|---|---|
| Buttersäuremethylester | 58,7 % |
| Buttersäure | 0,1 % |
| Wasser | 11,1 % |
| Sonstige | 0,1 % |

Im Reaktor verbleiben 51,8 g Sumpfprodukt, davon sind 50,5 g Ester. Die Ausbeute an Buttersäuremethylester beträgt 99,5 % der Theorie.

Beispiel 16

Herstellung von Isobuttersäuremethylester

Entsprechend Beispiel 1 wird in einen Kolben zu 200 g Biphenyl und 5,0 g konzentrierte Schwefelsäure stündlich ein Gemisch aus 102,0 g (1,16 mol) Isobuttersäure und 80,1 g (2,5 mol) Methanol bei einer Reaktionstemperatur von 120°C in den Reaktionskolben eindosiert. Insgesamt werden innerhalb von 7 Stunden 1274,7 g Ausgangsstoffgemisch eingesetzt. Im gleichen Zeitraum destillieren 1265,8 g Produkt über, das homogene Destillat hat folgende Zusammensetzung:

| Methanol | 24,5 % |
|---|---|
| Isobuttersäuremethylester | 63,0 % |
| Isobuttersäure | 1,0 % |
| Wasser | 11,1 % |
| Sonstige | 0,4 % |

Im Reaktor verbleiben 8,9 g Sumpfprodukt. Die Ausbeute an Isobuttersäuremethylester beträgt 96,4 % der Theorie.

Beispiel 17

Herstellung von Valeriansäuremethylester

Entsprechend Beispiel 1 wird in einen Kolben zu 200 g Biphenyl und 5,0 g konzentrierte Schwefelsäure stündlich ein Gemisch aus 102,0 g (1,0 mol) Valeriansäure und 80,1 g (2,5 mol) Methanol bei einer Reaktionstemperatur von 140°C eindosiert. Insgesamt werden innerhalb von 7 Stunden 1274,7 g Ausgangsstoffgemisch eingesetzt. Im gleichen Zeitraum destillieren 1095,5 g Produkt über, das homogene Destillat hat folgende Zusammensetzung:

| Methanol | 31,2 % |
|---|---|
| Valeriansäuremethylester | 57,0 % |
| Wasser | 11,2 % |
| Sonstige | 0,6 % |

Im Reaktor verbleiben 179,2 g Sumpfprodukt, davon sind 167,0 g Ester. Die Ausbeute an Valeriansäuremethylester beträgt 97,4 % der Theorie.

Beispiel 18

Herstellung von 2-Methylbuttersäuremethylester

Entsprechend Beispiel 1 wird in einen Kolben zu 200 g Biphenyl und 5,0 konzentrierte Schwefelsäure stündlich ein Gemisch aus 102,0 g (1,0 mol) 2-Methylbuttersäure und 80,1 g (2,5 mol) Methanol bei einer Reaktionstemperatur von 130°C eindosiert. Insgesamt werden innerhalb von 7 Stunden 1274,7 g Ausgangsstoffgemisch eingesetzt. Im gleichen Zeitraum destillieren 1145,3 g Produkt über, das homogene Destillat hat folgende Zusammensetzung:

| Methanol | 29,9 % |
|---|---|
| 2-Methylbuttersäuremethylester | 59,2 % |
| 2-Methylbuttersäure | 0,1 % |
| Wasser | 10,7 % |
| Sonstige | 0,1 % |

Im Reaktor verbleiben 129,4 g Sumpfprodukt, davon sind 110,4 g Ester. Die Ausbeute an 2-Methylbuttersäuremethylester beträgt 97,1 % der Theorie.

Beispiel 19

Herstellung von 3-Methylbuttersäuremethylester

Entsprechend Beispiel 1 wird in einen Kolben zu 200 g Biphenyl und 5,0 g konzentrierte Schwefelsäure stündlich ein Gemisch aus 102,0 g (1,0 mol) 3-Methylbuttersäure und 80,1 g (2,5 mol) Methanol bei einer Reaktionstemperatur von 130°C eindosiert. Insgesamt werden innerhalb von 7 Stunden 1274,7 g Ausgangsstoffgemisch eingesetzt. Im gleichen Zeitraum destillieren 1139,4 g Produkt über, das homogene Destillat hat folgende Zusammensetzung:

| | |
|---|---|
| Methanol | 29,9 % |
| 3-Methylbuttersäuremethylester | 58,9 % |
| 3-Methylbuttersäure | 0,1 % |
| Wasser | 11,0 % |
| Sonstige | 0,1 % |

Im Reaktor verbleiben 135,3 g Sumpfprodukt, davon sind 123,9 g Ester. Die Ausbeute an 3-Methylbuttersäuremethylester beträgt 97,9 % der Theorie.

Beispiel 20

Herstellung von Acrylsäuremethylester

Entsprechend Beispiel 1 wird in einen Kolben zu 200 g Biphenyl und 5,0 g konzentrierter Schwefelsäure stündlich ein Gemisch aus 72,0 g (1 mol) Acrylsäure und 80,1 g (2,5 mol) Methanol bei einer Reaktionstemperatur von 120°C eindosiert. Insgesamt werden innerhalb von 6 Stunden 912,6 g Ausgangsstoffgemisch eingesetzt. Im gleichen Zeitraum destillieren 839,3 g Produkt über, das homogene Destillat hat folgende Zusammensetzung:

| | |
|---|---|
| Methanol | 38,5 % |
| Acrylsäuremethylester | 50,5 % |
| Acrylsäure | <0,1 % |
| Wasser | 10,6 % |
| Sonstige | 0,4 % |

Im Reaktor verbleiben 73,3 g Sumpfprodukt, davon sind 16,6 g Ester. Die Ausbeute an Acrylsäuremethylester beträgt 85,1 % der Theorie.

Beispiel 21

Herstellung von Methacrylsäuremethylester

Entsprechend Beispiel 1 wird in einen Kolben zu 200 g Biphenyl und 5,0 g konzentrierter Schwefelsäure stündlich ein Gemisch aus 86,0 g (1 mol) Methacrylsäure und 80,1 g (2,5 mol) Methanol bei einer Reaktionstemperatur von 120°C eindosiert. Insgesamt werden innerhalb von 6 Stunden 996,6 g Ausgangsstoffgemisch eingesetzt. Im gleichen Zeitraum destillieren 941,0 g Produkt über, das homogene Destillat hat folgende Zusammensetzung:

| | |
|---|---|
| Methanol | 33,0 % |
| Methacrylsäuremethylester | 56,7 % |
| Methacrylsäure | <0,1 % |
| Wasser | 10,2 % |
| Sonstige | 0,1 % |

Im Reaktor verbleiben 55,6 g Sumpfprodukt, davon sind 12,4 g Ester. Die Ausbeute an Methacrylsäuremethylester beträgt 90,9 % der Theorie.

T a b e l l e   1

| Bei-spiel | Einsatz-mengen (g/h) | | Reakti-ons-dauer (h) | T (°C) | Destillat org. Ph. (g) | Zusammensetzung des Destillats org. Ph. (%) | | | | |
|---|---|---|---|---|---|---|---|---|---|---|
| | Pivalin-säure | MeOH | | | | $H_2O$ | MeOH | Ester | Säure | Rest |
| 2 | 102,1 | 96,0 | 6 | 120 | 1149,4 | 9,2 | 33,6 | 56,9 | 0,1 | 0,2 |
| 3 | 102,1 | 64,1 | 21 | 120 | 2959,7 | 6,3 | 17,3 | 76,4 | <0,1 | – |
| 4 | 102,1 | 56,1 | 20 | 120 | 2570,7 | 4,9 | 12,0 | 82,4 | 0,5 | 0,2 |

| | Destillat $H_2O$-Ph. | | Sumpf | | Ausbeute (% d.Th.) |
|---|---|---|---|---|---|
| | (g) | (% Ester) | (g) | (% Ester) | |
| 2 | – | – | 39,2 | 77,9 | 98,1 |
| 3 | 391,2 | 8,8 | 139,3 | 77,9 | 98,4 |
| 4 | 442,1 | 5,7 | 151,2 | 77,9 | 97,3 |

EP 0 286 981 B1

Tabelle 2

| Bei-spiel | Einsatz-mengen (g/h) | | Reakti-ons-dauer (h) | T (°C) | Destillat org. Ph. (g) | Zusammensetzung des Destillats org. Ph. (%) | | | | |
|---|---|---|---|---|---|---|---|---|---|---|
| | Pivalin-säure | MeOH | | | | $H_2O$ | MeOH | Ester | Säure | Rest |
| 5 | 102,1 | 96,0 | 6 | 110 | 1101,5 | 9,4 | 35,3 | 55,1 | 0,1 | 0,1 |
| 6 | 102,1 | 96,0 | 6 | 120 | 1149,4 | 9,2 | 33,6 | 56,9 | 0,1 | 0,2 |
| 7 | 102,1 | 96,1 | 7 | 130 | 1357,4 | 9,2 | 33,2 | 57,5 | – | 0,1 |

| | Destillat $H_2O$-Ph. | | Sumpf | | Ausbeute (% d.Th.) |
|---|---|---|---|---|---|
| | (g) | (% Ester) | (g) | (% Ester) | |
| 5 | – | – | 87,1 | 77,9 | 96,8 |
| 6 | – | – | 39,2 | 77,9 | 98,1 |
| 7 | – | – | 30,0 | 86,3 | 99,2 |

EP 0 286 981 B1

T a b e l l e   3

| Bei-spiel | Einsatz-mengen (g/h) | | Reakti-ons-dauer (h) | T (°C) | Destillat org. Ph. (g) | Zusammensetzung des Destillats org. Ph. (%) | | | | |
|---|---|---|---|---|---|---|---|---|---|---|
| | Pivalin-säure | MeOH | | | | $H_2O$ | MeOH | Ester | Säure | Rest |
| 8 | 136,1 | 106,8 | 4,5 | 120 | 1042,4 | 9,5 | 29,0 | 58,7 | 1,3 | 1,5 |
| 9 | 94,2 | 73,9 | 6 | 120 | 994,9 | 9,9 | 26,9 | 62,9 | 0,2 | 0,1 |

| | Destillat $H_2O$-Ph. | | Sumpf | | Ausbeute (% d.Th.) |
|---|---|---|---|---|---|
| | (g) | (% Ester) | (g) | (% Ester) | |
| 8 | – | – | 50,7 | 64,3 | 92,4 |
| 9 | – | – | 13,7 | 74,8 | 98,9 |

EP 0 286 981 B1

Tabelle 4

| Bei-spiel | Einsatzmengen (g/h) | | Reaktions-dauer (h) | Kataly-sator-art | Destillat org. Ph. (g) | Zusammensetzung des Destillats org. Ph. (%) | | | | |
|---|---|---|---|---|---|---|---|---|---|---|
| | Pivalin-säure | MeOH | | | | $H_2O$ | MeOH | Ester | Säure | Rest |
| 10 | 94,2 | 73,9 | 6 | $H_2SO_4$ | 994,9 | 9,9 | 26,9 | 62,9 | 0,2 | 0,1 |
| 11 | 102,1 | 80,0 | 7 | $NaHSO_4$ | 1084,9 | 9,3 | 34,9 | 54,4 | 1,3 | 0,1 |
| 12 | 94,1 | 73,9 | 51,5 | pTSS | 8555,9 | 9,9 | 26,9 | 62,8 | 0,2 | 0,2 |

| Bei-spiel | Sumpf | | Ausbeute (% d.Th.) |
|---|---|---|---|
| | (g) | (% Ester) | |
| 10 | 13,7 | 74,8 | 98,9 |
| 11 | 189,8 | 34,6 | 80,6 |
| 12 | 96,1 | 77,9 | 98,7 |

## Patentansprüche

1. Verfahren zur Herstellung von Carbonsäuremethylestern aus gesättigten oder ungesättigten, geradkettigen oder verzweigten Monocarbonsäuren mit 1 bis 5 Kohlenstoffatomen im Molekül und Methanol, dadurch gekennzeichnet, daß man Carbonsäure und Methanol im Molverhältnis von 1 : 1 bis 1 : 5 getrennt oder als Mischung bei Temperaturen oberhalb des Siedepunktes des resultierenden Esters, mindestens aber bei 110°C in ein Gemisch aus einem sauren Katalysator und einem unter den Reaktionsbedingungen flüssigen, gegenüber den Reaktanten und den Reaktionsprodukten inerten Wärmeträger unter Einhaltung einer Verweilzeit von 0,1 bis 5, insbesondere 0,5 bis 2 Stunden leitet.

2.  Verfahren nach Anspruch 1, dadurch gekennzeichnet, daß der Wärmeträger mindestens 50°C oberhalb der zu veresternden Säure siedet.

3.  Verfahren nach Anspruch 1 oder 2, dadurch gekennzeichnet, daß der Wärmeträger Biphenyl ist.

4.  Verfahren nach einem oder mehreren der Ansprüche 1 bis 3, dadurch gekennzeichnet, daß als Veresterungskatalysator Schwefelsäure eingesetzt wird.

5.  Verfahren nach einem oder mehreren der Ansprüche 1 bis 4, dadurch gekennzeichnet, daß die Reaktionstemperatur 10 bis 100°C oberhalb des Siedepunktes des resultierenden Esters liegt.

6.  Verfahren nach einem oder mehreren der Ansprüche 1 bis 5, dadurch gekennzeichnet, daß das Molverhältnis von Carbonsäure zu Methanol 1 : 1,75 bis 1 : 3 beträgt.

**Claims**

1.  A process for the preparation of carboxylic acid methyl esters from saturated or unsaturated, straight-chain or branched monocarboxylic acids having 1 to 5 carbon acids in the molecule and methanol, characterised in that carboxylic acid and methanol are passed in a molar ratio of 1 : 1 to 1 : 5 either separately or as a mixture into a mixture of an acidic catalyst and a heat transfer medium, which is liquid under the reaction conditions and inert to the reactants and the reaction products, at temperatures above the boiling point of the resultant ester but not less than 110°C, a residence period of 0.1 to 5, in particular 0.5 to 2 hours being observed.

2.  A process according to claim 1, characterised in that the boiling point of heat transfer medium is at least 50°C above the boiling point of the acid to be esterified.

3.  A process according to claims 1 or 2, characterised in that the heat transfer medium is biphenyl.

4.  A process according to one or more of the claims 1 to 3, characterised in that sulfuric acid is used as the esterification catalyst.

5.  A process according to one or more of the claims 1 to 4, characterised in that the reaction temperature is 10 to 100°C above the boiling point of the resultant ester.

6.  A process according to one or more of the claims 1 to 5, characterised in that the molar ratio of the carboxylic acid to the methanol is 1 : 1.75 to 1:3.

**Revendications**

1.  Procédé de préparation d'esters méthyliques d'acides carboxyliques à partir d'acides monocarboxyliques saturés ou insaturés à chaîne droite ou ramifiée contenant 1 à 5 atomes de carbone dans la molécule et du méthanol, caractérisé en ce que l'on envoie l'acide carboxylique et le méthanol à un rapport molaire de 1:1 à 1:5, séparément ou à l'état de mélange, à des températures supérieures au point d'ébullition de l'ester formé mais à 110°C au moins, dans un mélange d'un catalyseur acide et d'un agent de transfert de la chaleur liquide dans les conditions de la réaction, inerte à l'égard des réactifs et des produits de réaction, en maintenant une durée de passage de 0,1 à 5, de préférence de 0,5 à 2 h.

2.  Procédé selon la revendication 1, caractérisé en ce que l'agent de transfert de la chaleur bout à au moins 50°C au-dessus de l'acide à estérifier.

3.  Procédé selon la revendication 1 ou 2, caractérisé en ce que l'agent de transfert de la chaleur est le biphényle.

4. Procédé selon une ou plusieurs des revendications 1 à 3, caractérisé en ce que l'on utilise l'acide sulfurique en tant que catalyseur d'estérification.

5. Procédé selon une ou plusieurs des revendications 1 à 4, caractérisé en ce que la température de réaction est supérieure de 10 à 100°C à la température d'ébullition de l'ester formé.

6. Procédé selon une ou plusieurs des revendications 1 à 5, caractérisé en ce que le rapport molaire acide carboxylique/méthanol est de 1:1,75 à 1:3.